# EUROPÄISCHE PATENTANMELDUNG

(11) **EP 2 578 096 A1**
(43) Veröffentlichungstag der Anmeldung: **10.04.2013**
(21) Anmeldenummer: 11075222.7
(22) Anmeldetag: 07.10.2011
(51) Int. Cl.: A41D 19/015, H01H 13/702, A61F 7/00

(54) **Kleidungsteil mit einem Bedienteil**

(71) Anmelder: Sidas Central GmbH in Gründung, 1010 Wien (AT)
(72) Erfinder: Kremer, Gerhard, 8280 Fürstenfield (AT); Maron, Urs, 2560 Nidau (CH)
(74) Vertreter: Pfenning, Meinig & Partner GbR

(57) **Zusammenfassung**

Kleidungsteil (1) mit einem Bedienteil (2) zum manuellen Bedienen einer mit dem Kleidungsteil (1) verbindbaren, steuerbaren Vorrichtung (24) mittels mindestens eines Bedienelementes (3, 4) des Bedienteils, wobei das Bedienteil (2) ein erstes Formteil (5) umfasst, welches fest mit dem Kleidungsteil (1) verbunden ist und welches das mindestens eine Bedienelement (3, 4) aufweist, und ein zweites Formteil (13), das eine Aufnahme (14) für eine Steuereinheit (18) der Vorrichtung (24) aufweist und mit dem ersten Formteil.(5) verbindbar ist, wobei das erste Formteil (5) mindestens ein erstes Rückhalteelement (9) aufweist und dass das zweite Formteil (13) mindestens ein zweites Rückhalteelement (15) aufweist, wobei die beiden Rückhalteelemente (13, 15) in einen gegenseitigen Eingriff bringbar sind zum Herstellen einer lösbaren Verbindung zwischen dem ersten Formteil (5) und dem zweiten Formteil (13).

## Beschreibung

Gegenstand der Erfindung ist ein Kleidungsteil nach den Merkmalen des Oberbegriffs des Anspruchs 1.

Derartige gattungsgemäße Kleidungsteile mit steuerbaren Vorrichtungen sind aus der Druckschrift WO 2009/092618 A1 bekannt. Darin wird ein solches Kleidungsteil in einem Ausführungsbeispiel in der Form eines Handschuhs mit einer Heizvorrichtung beschrieben. Zum manuellen Bedienen ihrer steuerbaren Vorrichtung weist ein gattungsgemäßes Kleidungsteil ein Bedienteil auf mit einem oder mehreren Bedienelementen, wie beispielsweise eine oder mehrere Drucktasten. Über das mindestens eine Bedienelement ist eine Steuereinheit der Vorrichtung bedienbar werden, um Steuerparameter zum Steuern der Vorrichtung einzustellen, wie etwa eine gewünschte Heizleistung.

Wie in der Druckschrift WO 2009/092618 A1 beschrieben, kann diese Steuereinheit beispielsweise eine Leiterplatte mit einer elektrischen Steuerschaltung umfassen, die beispielsweise zur Regelung der Temperatur eines Wärmeübertragungselements der Vorrichtung eingerichtet sein kann.

Um die Steuereinheit vor äußeren Einflüssen zu schützen, ist es im Stand der Technik vorgesehen, die Steuereinheit innerhalb einer Umkapselung des Bedienteils anzuordnen, welche fest mit dem Kleidungsteil verbunden ist. Durch ein solches Bedienteil lässt sich eine Vorrichtung eines Kleidungsteils zwar komfortabel bedienen, jedoch stellt sich die Reinigung solcher Kleidungsteile als problematisch heraus. Es besteht nämlich die Gefahr, dass beim Reinigen des Kleidungsteils Wasser oder Reinigungsmittel in das Bedienteil eintritt und die Steuereinheit beschädigt. Besonders die Stelle, an der eine Steuerleitung durch die Umkapselung der Steuereinheit hindurchtritt zum Übertragen von Steuersignalen von der Steuereinheit auf die Vorrichtung, stellt diesbezüglich eine Schwachstelle dar, da sich besonders an dieser Stelle leicht Undichtigkeiten ergeben können.

Es ist also die Aufgabe der vorliegenden Erfindung, ein Kleidungsteil mit einem Bedienteil zum manuellen Bedienen einer mit dem Kleidungsteil verbindbaren, steuerbaren Vorrichtung vorzuschlagen, welche die beschriebenen Nachteile des Standes der Technik überwindet. Ein solches Kleidungsteil soll also leicht zu reinigen sein, ohne dass dabei die Gefahr einer Beschädigung der Steuereinheit der Vorrichtung besteht, etwa durch in das Bedienteil und die Steuereinheit eintretende Flüssigkeiten.

Diese Aufgabe wird erfindungsgemäß gelöst durch ein Kleidungsteil gemäß dem Hauptanspruch. Weiterentwicklungen und spezielle Ausführungsformen der Erfindung sind Gegenstände der abhängigen Ansprüche.

Bei einem erfindungsgemäßen Kleidungsteil mit einem Bedienteil zum manuellen Bedienen einer mit dem Kleidungsteil verbindbaren, steuerbaren Vorrichtung mittels mindestens eines Bedienelementes des Bedienteils, umfasst das Bedienteil demnach ein erstes Formteil, welches fest mit dem Kleidungsteil verbunden ist und welches das mindestens eine Bedienelement aufweist. Außerdem umfasst das Bedienteil ein zweites Formteil, das eine Aufnahme, wie beispielsweise eine Vertiefung oder ein Schubfach, für eine Steuereinheit der Vorrichtung aufweist und das mit dem ersten Formteil verbindbar ist.

Für die Erfindung ist es nun wesentlich, dass das erste Formteil mindestens ein erstes Rückhalteelement aufweist und dass das zweite Formteil mindestens ein zweites Rückhalteelement aufweist, wobei die beiden Rückhalteelemente in einen gegenseitigen Eingriff bringbar sind zum Herstellen einer lösbaren Verbindung zwischen dem ersten Formteil und dem zweiten Formteil. Sobald mittels dieser Rückhalteelemente also die beiden Formteile miteinander verbunden sind, ist das zweite Formteil auch mit dem Kleidungsstück verbunden, indem es mittels der Rückhalteelemente an dem ersten Formteil, welches fest mit dem Kleidungsstück verbunden ist, beispielsweise durch Vernähen, festgehalten wird. Nach Lösen des gegenseitigen Eingriffs der Rückhalteelemente kann das zweite Formteil von dem ersten Formteil und somit auch von dem Kleidungsteil gelöst und entfernt werden.

Gleichzeitig kann die Steuereinheit durch das erste und das zweiten Formteil umkapselt und so vor sicher fixiert und vor äußeren Einflüssen geschützt werden, wenn das erste und das zweite Formteil miteinander verbunden sind. Die beiden Formteile bilden dann also eine Umkapselung für die Steuereinheit, die jedoch einfach und problemlos geöffnet werden kann, etwa um die Steuereinheit zu entfernen oder durch eine andere auszutauschen.

Beispielsweise kann eines des mindestens einen ersten Rückhalteelementes als eine das erste Formteil vorzugsweise vollständig umlaufende Krempe ausgestaltet sein. Es ist aber auch möglich, dass eines des mindestens einen zweiten Rückhalteelements als eine das zweite Formteil vollständig umlaufende Krempe ausgestaltet ist. Ebenso ist es möglich, dass eines des mindestens einen ersten Rückhalteelements als eine Außenkante des ersten Formteils ausgestaltet ist oder dass eines des mindestens einen zweiten Rückhalteelements als eine Außenkante des zweiten Formteils ausgestaltet ist. Entscheidend für die spezielle Ausgestaltung der genannten Rückhalteelemente ist es, dass diese so ineinander eingreifen können, dass bei Bestehen des Eingriffs eine lösbare, formschlüssige Verbindung zwischen dem erste Formteil und dem zweiten Formteil hergestellt ist. Dies kann beispielsweise dadurch gegeben sein, dass das mindestens eine erste (bzw. zweite) Rückhalteelement das mindestens eine zweite (bzw. erste) Rückhalteelement hintergreift, sobald und so lange eine solche Verbindung zwischen den beiden Formteilen besteht.

Zum Herstellen oder Lösen einer solchen Verbindung zwischen den beiden Formteilen kann es vorgesehen sein, dass zunächst zumindest eines der Rückhalteelemente oder alle Rückhalteelemente aus einer jeweiligen Gleichgewichtsposition heraus bewegt werden, um den Formschluss und den Eingriff zwischen den Rückhalteelementen herzustellen bzw. zu lösen.

Falls sich also in der Aufnahme des zweiten Formteils eine Steuereinheit einer mit dem Kleidungsstück verbindbaren oder verbundenen Vorrichtung befindet, kann die Steuereinheit durch Lösen des Eingriffs zwischen den beiden Rückhalteelementen und durch Lösen der Verbindung zwischen dem ersten und dem zweiten Formteil die Steuereinheit von dem Kleidungsstück problemlos getrennt werden, beispielsweise um anschließend das Kleidungsstück reinigen oder waschen zu können. Natürlich ist es auf diese Weise auch möglich, die Steuereinheit gegen eine andere Steuereinheit auszutauschen, beispielsweise im Fall eines Defekts der Steuereinheit. Möglicherweise ist die Steuereinheit über mindestens eine elektrische Leitung, wie etwa eine Steuerleitung zum Übertragen von Steuersignalen, mit der Vorrichtung verbindbar, wie weiter unten beschrieben wird. Dann kann außerdem mindestens eine Steckverbindung vorgesehen sein zum Herstellen und Lösen einer Verbindung zwischen der mindestens Leitung und der Vorrichtung und/oder zwischen der Leitung und der Steuereinheit.

In einer Weiterentwicklung ist vorgesehen, dass die Aufnahme des zweiten Formteils für die Steuereinheit als eine Vertiefung in dem zweiten Formteil ausgestaltet ist. Diese Vertiefung kann beispielsweise mit einer Schutzfolie oder einem Schutzdeckel abgedeckt sein. Auf diese Weise kann eine sich in der Vertiefung befindliche Steuereinheit vor äußeren Einflüssen geschützt werden, beispielsweise vor Feuchtigkeit, Spritzwasser, Verschmutzungen oder mechanische Einwirkungen. Außerdem kann durch eine solche Schutzfolie oder einen solchen Schutzdeckel verhindert werden, dass sich die Steuereinheit ungewollt von dem zweiten Formteil löst und dabei möglicherweise verlorengeht. Insgesamt lässt sich also mittels einer solchen Schutzfolie oder einem solchen Schutzdeckel eine besonders sichere Aufbewahrung der Steuereinheit gewährleisten, beispielsweise während einer Reinigung des Kleidungsteils oder einem Austausch der Steuereinheit.

In einer Weiterentwicklung ist vorgesehen, dass das erste Formteil einen flanschförmigen ersten Randbereich aufweist und dass das zweite Formteil einen flanschförmigen zweiten Randbereich aufweist. Wenn und sobald die beiden Formteile miteinander verbunden sind (durch den oben beschriebenen gegenseitigen Eingriff der Rückhalteelemente der beiden Formteile) liegen diese flanschförmigen Randbereiche flächig aufeinander und umlaufen die Aufnahme für die Steuereinheit vollständig, d.h. ohne Unterbrechungen. Die beiden Randbereiche werden vorzugsweise außerdem durch eine permanente Anpresskraft aufeinandergedrückt, welche durch die beiden Rückhalteelemente hervorgerufen wird. Ein solches flächiges Aufeinanderliegen und gegebenenfalls auch ein Aufeinanderpressen der beiden flanschförmigen Randbereiche bewirkt eine besonders sichere Abdichtung der Aufnahme der Steuereinheit, so dass diese besonders zuverlässig geschützt ist, beispielsweise vor einem Flüssigkeitseintritt. Zum Herstellen der genannten Anpresskraft sind alle oder zumindest manche der Rückhalteelemente elastisch ausgestaltet. Zu diesem Zweck können die betreffenden Rückhalteelemente aus einem flexiblen und dauerelastischen Material gefertigt werden, beispielsweise einem Kunststoff, wie etwa Silikon. Als Materialien für das Bedienteil, insbesondere für das erste und/oder zweite Formteil und/oder die Rückhalteelemente des ersten und/oder zweite Formteils, kommen alle gießfähigen, pressfähigen, extrudierbaren, spritzbaren und/oder blasformbaren Materialien in Frage, wie beispielsweise thermoplastische Elastomere (TPE), Thermoplastische (Poly)-Urethane (TPU), gummiartige Materialien. Die betreffenden Teile können aus diesen Materialen also gegossen, gepresst, extrudiert, gespritzt oder blasgeformt sein. Es ist natürlich auch möglich das erste und/oder das zweite Formteil vollständig aus einem solchen dauerelastischen Material zu fertigen. Die oben genannte Schutzfolie und der oben genannte Schutzdeckel können ebenfalls aus einem elastischen Material gefertigt sein, wobei auch hierfür die oben genannten Materialien in Frage kommen. Die Verwendung (dauer-)elastischer Materialen kann außerdem einen Tragekomfort des Bedienteils und des Kleidungsteils erhöhen. Außerdem kann durch die Verwendung elastischer Materialien erreicht werden, dass die Verbindung zwischen dem ersten Formteil und dem zweiten Formteil besonders einfach hergestellt und wieder gelöst werden kann.

In einer Weiterentwicklung ist vorgesehen, dass das zweite Formteil ein Loch aufweist zum Hindurchführen der bereits genannten elektrischen Leitungen, wie beispielsweise Steuerleitungen, durch das zweite Formteil hindurch. Auf diese Weise kann vermieden werden, dass genannten Leitungen durch eine Grenzfläche bzw. einen Grenzbereich zwischen dem ersten und dem zweiten Formteil hindurchgeführt werden müssen, so dass die Gefahr von Undichtigkeiten zwischen dem ersten und dem zweiten Formteil reduziert wird.

Die Gefahr von Undichtigkeiten kann auch mittels zusätzlicher Dichtungselemente reduziert werden, welche vorzugsweise ringförmig ausgestaltet sind und vorzugsweise den Aufnahmebereich für die Steuereinheit vollständig umlaufen. Beispielsweise kann ein solches Dichtungselement in Form eines Steges auf einem der beiden Formteile ausgestaltet oder durch einen separaten Dichtungsring gegeben sein.

In einer Weiterentwicklung ist vorgesehen, dass das Formteil zumindest bereichsweise durchsichtig ausgestaltet ist, dass es also zumindest in einem Bereich aus einem durchsichtigen Material besteht, um auf diese Weise die Sicht auf eine Anzeige der Steuereinheit durch das erste Formteil hindurch zu ermöglichen. Zu diesem Zweck kann auch die oben genannte Schutzfolie bzw. der oben genannte Schutzdeckel vollständig oder zumindest bereichsweise durchsichtig sein bzw. aus einem durchsichtigen Material gefertigt sein.

In einer speziellen Ausführungsform ist die Vorrichtung des Kleidungsteils durch eine Heizvorrichtung gegeben, wie sie beispielsweise in der eingangs genannten Druckschrift WO 2009/092618 A1 beschrieben wird.

Es kann ferner vorgesehen sein, dass die Steuereinheit mindestens eine Eingabeschnittstelle aufweist zur manuellen Eingabe von Steuerparametern in die Steuereinheit, wobei die mindestens eine Eingabeschnittstelle der Steuereinheit auf einer Oberseite der Steuereinheit so platziert ist, dass die mindestens eine Eingabeschnittstelle mittels des mindestens einen Eingabeelements des ersten Formteils bedient werden kann, wenn und sobald die beiden Formteile miteinander verbunden sind durch den beschriebenen gegenseitigen Eingriff der Rückhalteelemente der beiden Formteile. Im bereits genannten Beispiel, dass die Vorrichtung eine Heizvorrichtung ist, können zwei Eingabeschnittstellen vorgesehen sein, welche mittels zweier Eingabeelemente des ersten Formteils bedienbar sind, wobei eine erste der beiden Eingabeschnittstellen zur Erhöhung einer Heizleistung dient und die zweite der beiden Eingabeschnittstellen zur Reduzierung einer Heizleistung. Dabei ist die Steuereinheit dazu eingerichtet, je nach Wahl der Heizleistung, die Heizvorrichtung zur Abgabe dieser Heizleistung anzusteuern. Für eine spezielle Ausführung dieser Eingabeelemente und Eingabeschnittstellen sei wiederum auf die Druckschrift WO 2009/092618 A1 verwiesen.

Zur Übertragung von Steuersignalen kann die Steuereinheit über die bereits genannte mindestens eine Steuerleitung mit der Vorrichtung verbunden sein, wobei ein erstes Ende der Steuerleitung mit der Steuereinheit und ein zweites Ende der Steuerleitung mit der Vorrichtung verbunden ist. In einer Ausführungsform ist die mindestens eine Steuerleitung zweiteilig ausgeführt, wobei ein erster Teil der Steuerleitung mit der Steuereinheit verbunden ist und ein zweiter Teil der Steuerleitung mit der Vorrichtung verbunden ist. Beide Teile weisen jeweils ein Steckelement auf zum Verbinden der beiden Teile der Steuerleitung. Beim Entfernen der Steuereinheit von dem Kleidungsteil können dann die beiden Teile der mindestens einen Steuerleitung mittels der beiden Steckelemente getrennt werden, so dass der erste Teil der Steuerleitung mit der Steuereinheit verbunden bleibt und der zweite Teil der Steuerleitung mit der mit dem Kleidungsteil verbundenen Vorrichtung. Entsprechend können die beiden Teile der Steuerleitung mittels dieser Steckelemente auch wieder miteinander verbunden werden, sobald die Steuereinheit wieder mit dem Kleidungsteil verbunden werden soll. Es ist auch möglich, Verbindungsvorrichtungen für Steuerleitungen direkt an der steuerbare Vorrichtung und/oder an dem zweiten Formteils anzuordnen, so dass die mindestens eine Steuerleitung über derartige Verbindungsvorrichtungen, wie etwa Stecker oder Steckdosen, mit der Vorrichtung oder der Steuereinheit verbunden werden können.

Unter dem Kleidungsteil soll allgemein ein Bekleidungsstück oder auch eine Decke, ein Kissen oder ein Schlafsack verstanden werden. Bei dem Kleidungsteil kann es sich aber auch um einen Teil eines solchen Bekleidungsstücks handeln. Somit kann es sich bei dem Kleidungsteil beispielsweise um eine Jacke, eine Hose, einen Schuh, einen Handschuh, eine Socke oder eine Mütze handeln oder um einen Teil einer Jacke, einer Hose, eines Schuhs, eines Handschuhs, einer Socke oder einer Mütze. Beispielsweise kann es sich bei dem Kleidungsteil also auch um eine Einlegesohle eines Schuhs oder einer Innen- oder Außenhülle eines Schuhs, einer Jacke oder einer Hose handeln.

Im Folgenden wird die Erfindung anhand eines in den Figuren 1 bis 10c schematisch dargestellten speziellen Ausführungsbeispiels näher erläutert. Es zeigt:
- Fig. 1: ein Kleidungsteil hier vorgeschlagener Art mit einem Bedienteil zum manuellen Bedienen einer mit dem Kleidungsteil verbundenen, steuerbaren Heizvorrichtung,
- Fign. 2 bis 4b: ein erstes Formteil eines Bedienteils eines Kleidungsteils hier vorgeschlagener Art in verschiedenen Ansichten,
- Fign. 5a bis 8: ein zweites Formteil eines Bedienteils eines Kleidungsteils hier vorgeschlagener Art in verschiedenen Ansichten,
- Fig. 9: die in Fig. 8 gezeigte Schutzabdeckung in eiern Einzelansicht,
- Fign. 10a bis 10c: ein erstes Formteil und ein zweites Formteil eines Bedienteils eines Kleidungsteils hier vorgeschlagener Art vor bzw. nach Herstellung einer formschlüssigen Verbindung zwischen den beiden Formteilen.

Wiederkehrende Merkmale werden mit den gleichen Bezugszeichen versehen.

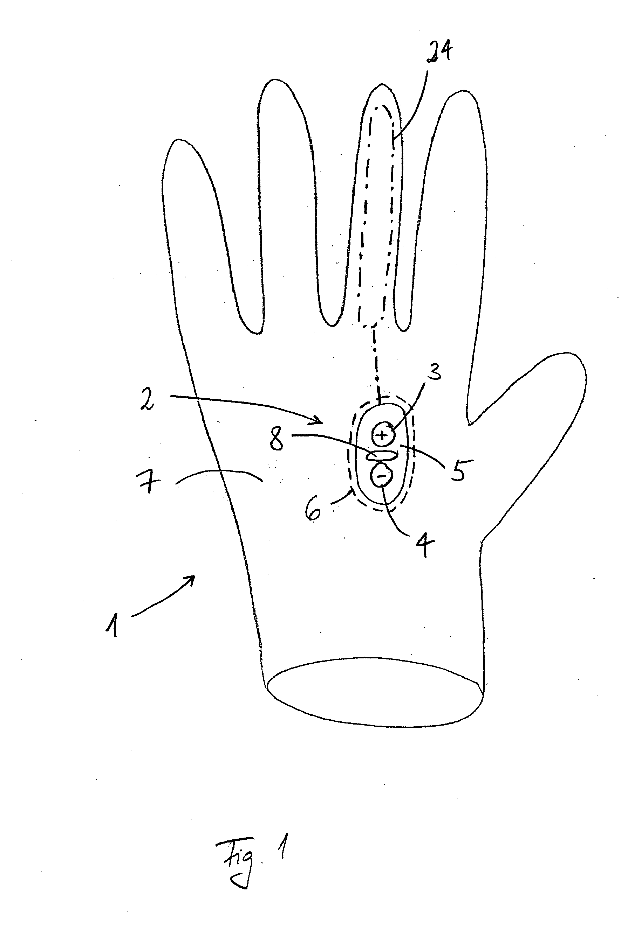

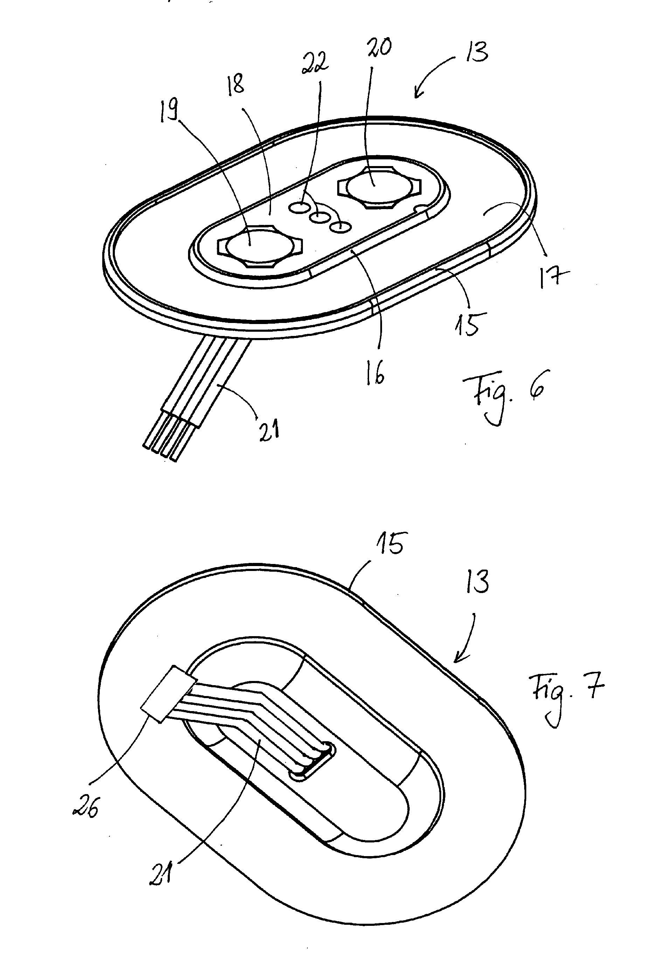

In Fig. 1 ist ein spezielles Ausführungsbeispiel eines Kleidungsteils 1 hier vorgeschlagener Art schematisch dargestellt. Bei dem Kleidungsteil 1 handelt es sich um einen Handschuh mit einem Bedienteil 2 zum manuellen Bedienen einer mit dem Kleidungsteil 1 verbundenen, steuerbaren Heizvorrichtung 24 mittels einem ersten und einem zweiten Bedienelement 3, 4. Die Heizvorrichtung 24 innerhalb des Kleidungsteils 1 ist der besseren Übersichtlichkeit halber nur schematisch durch eine Strichpunktlinie angedeutet. Für Details zu möglichen Ausführungsformen der Heizvorrichtung 24 wird an dieser Stelle auf den Stand der Technik verwiesen, beispielsweise auf die Druckschrift WO 2009/092618 A1. Die beiden Bedienelemente 3, 4 sind auf einem ersten Formteil 5 des Bedienteils angeordnet, das mittels einer Naht 6 mit einer Außenhülle 7 des Kleidungsteils 1 fest verbunden ist und einen transparenten Bereich 8 aufweist, durch den eine Anzeige einer Steuereinheit, vgl. Fig. 6, sichtbar ist.

Bei dem gezeigten Kleidungsteil 1 könnte es sich auch um eine Jacke, eine Hose, einen Schuh, eine Socke, eine Mütze oder ein anderes Bekleidungsstück handeln. Das Kleidungsteil könnte aber auch eine Decke, ein Kissen oder ein Schlafsack sein.

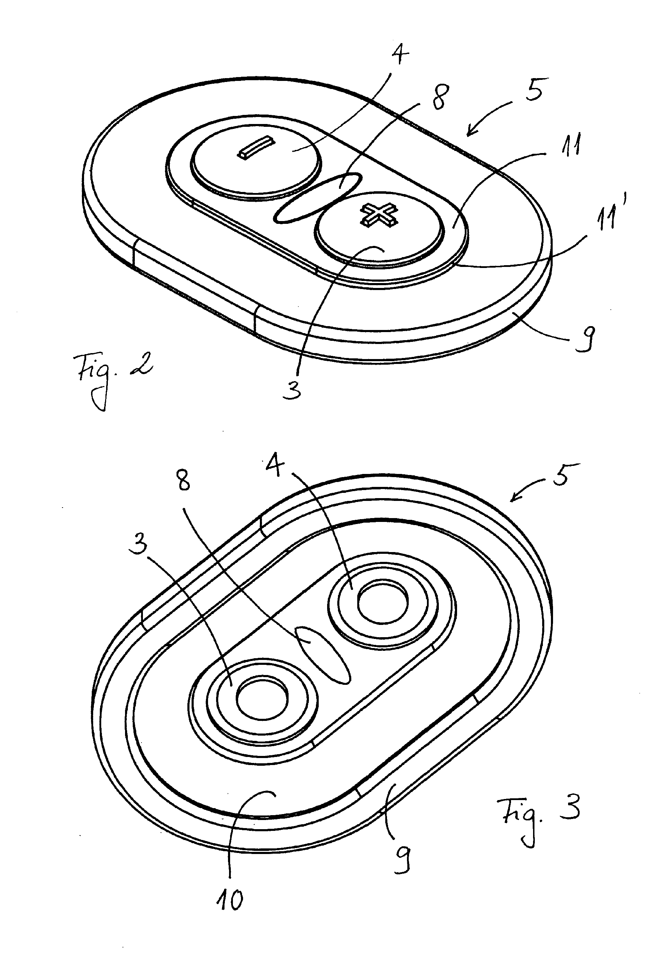

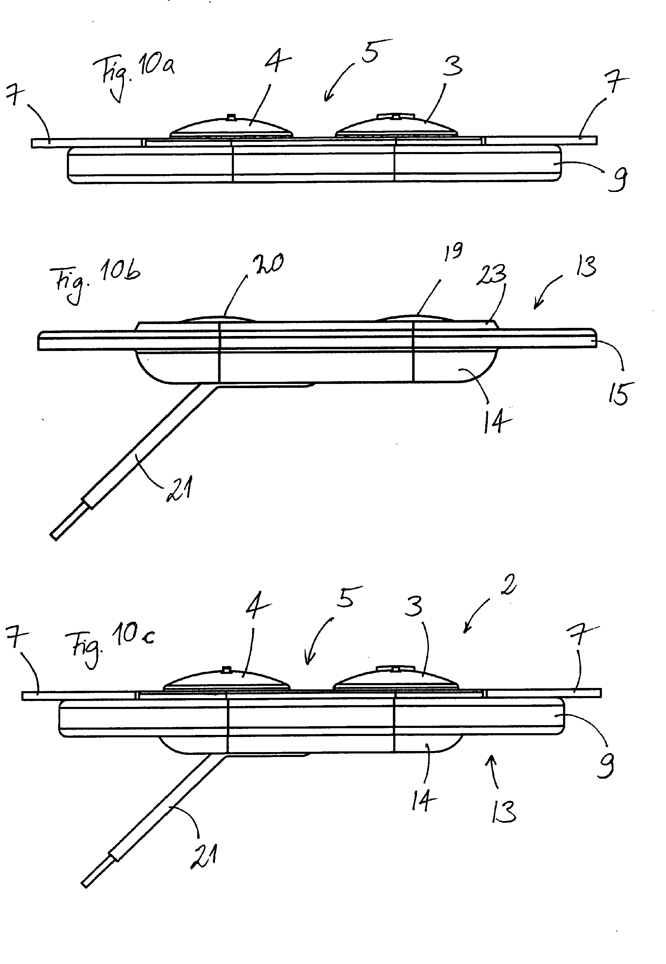

In Fig. 2 ist ein erstes Formteil 5 eines Bedienteils eines Kleidungsteils hier vorgeschlagener Art schematisch dargestellt. Hierbei könnte es sich beispielsweise um das in Fig. 1 gezeigte erste Formteil 5 handeln. Deutlich zu erkennen sind ein erstes und ein zweites Bedienelement 3 und 4, die als formflexible Druckknöpfe ausgestaltet sind und durch manuelles Bedienen eingedrückt werden können, beispielsweise um Steuerparameter einer Steuereinheit, die direkt unterhalb der Bedienelemente 3, 4 angeordnet sein kann, vgl. Fign. 10a-10c, einzustellen. Zwischen den beiden Bedienelementen 3, 4 ist ein transparenter Bereich 8 angeordnet, durch den hindurch eine Anzeige einer solchen Steuereinheit sichtbar ist.

Das gezeigte erste Formteil 5 weist ein erstes Rückhalteelement 9 in Form eines Kragens auf, der das erste Formteil 5 vollumfänglich umläuft. Das erste Formteil 5 ist vollständig aus einem elastischen Material gefertigt, welches in diesem Beispiel durch Silikon gegeben ist.

In Fig. 3 ist das in Fig. 2 gezeigte erste Formteil 5 in einer Ansicht von schräg unten schematisch dargestellt. Zu erkennen sind insbesondere Unterseiten der beiden Bedienelemente 3, 4 sowie der transparente Bereich 8. Zu sehen ist außerdem das als Krempe ausgestaltete und das erste Formteil 5 vollständig umlaufende erste Rückhalteelement 9. Man erkennt ferner einen flächigen, flanschförmigen erster Randbereich 10 des ersten Formteils.

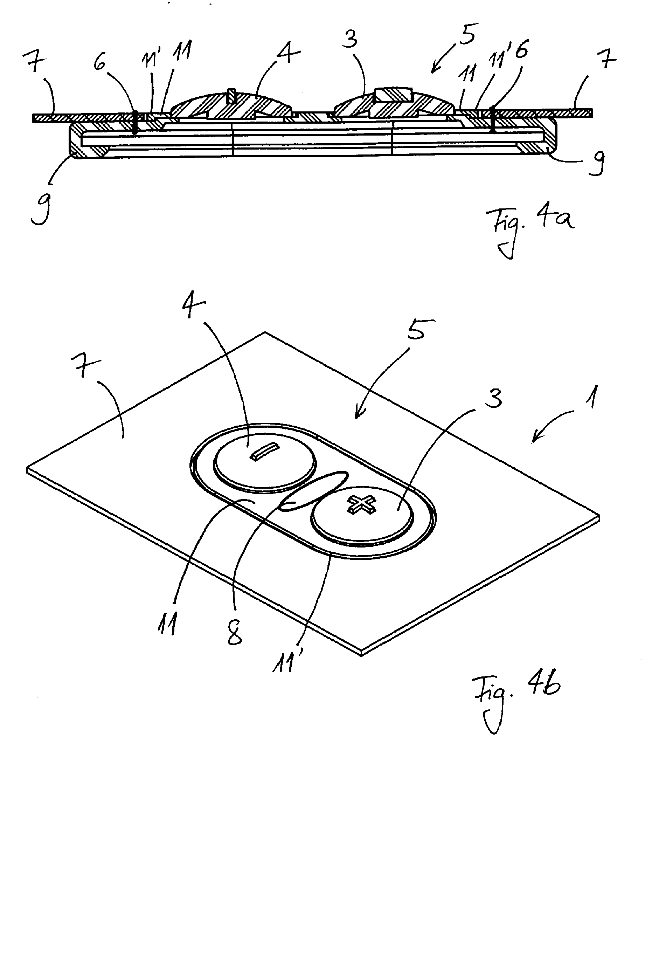

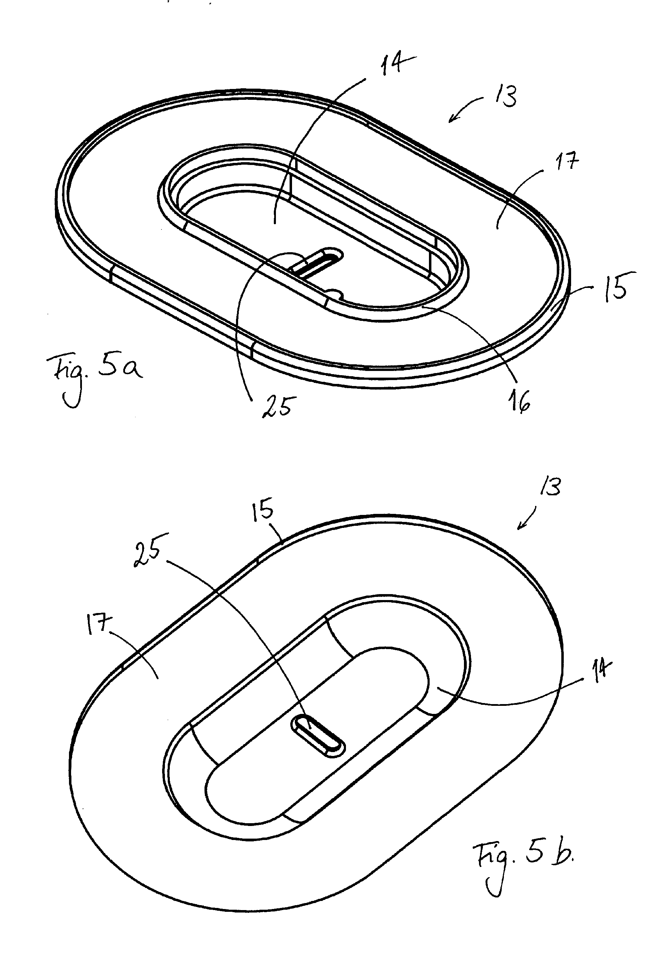

In Fig. 4a ist ein Querschnitt durch das in den Fign. 2 und 3 gezeigte erste Formteil 5 schematisch dargestellt. In dieser Darstellung sind das erste, krempenförmige Rückhalteelement 9 zu sehen sowie der flanschförmige Randbereich 10, eines der beiden Bedienelemente 3, 4 sowie eine Außenhülle 7 eines Kleidungsteils 1 hier vorgeschlagener Art, wie beispielsweise der in Fig. 1 gezeigte Handschuh, und eine Naht 6, der das erste Formteil 5 mit der Außenhülle 7 des Kleidungsteils 1 fest verbindet. Die Naht 6 umläuft dabei, wie im in Fig. 1 gezeigten Beispiel, die Bedienelemente 3, 4 wie auch den transparenten Bereich (hier nicht sichtbar). Die beiden Bedienelemente, der transparente Bereich sind auf einem Trägerelement 11 des ersten Formteils 5 angeordnet, das gegenüber dem ersten Randbereich 10 erhöht ist, so dass zwischen dem Trägerelement 11 und dem ersten Randbereich 10 eine Stufe 11' mit einer Höhe von etwa 1 mm ausgebildet ist, so dass die Außenhülle 7 des Kleidungsteils 1 mit dem Trägerelement 11 bündig abschließt. Auf einer Unterseite des ersten Formteils 5 ist direkt unterhalb des Trägerelements 11 eine Ausnehmung 12 eingearbeitet zur Aufnahme eines Dichtungselements des zweiten Formteils, vgl. Fign. 5a und 5b.

In Fig. 4b ist das in Fig. 4a gezeigte erste Formteil 5 noch einmal in einer Ansicht von schräg oben schematisch dargestellt, wobei hier insbesondere zu erkennen ist, dass die Außenhülle 7 des Kleidungsteils 1 (letzteres hier nur ausschnittsweise zu sehen) mit dem Trageelement 11 des ersten Formteils 5 bündig abschließt aufgrund der Stufe 11' des Trageelements 11. Die Stufe 11' des Trageelements 11 sind außerdem auch in Figur 2 zu sehen.

Fign. 5a und 5b zeigen jeweils schematisch von schräg oben bzw. von schräg unten ein zweites Formteil 13 eines Bedienteils eines Kleidungsteils hier vorgeschlagener Art, beispielsweise des in Fig. 1 dargestellten Kleidungsteils 1, mit einer Aufnahme 14 für eine Steuereinheit (hier nicht dargestellt, vgl. Fig. 6) einer steuerbaren Vorrichtung des Kleidungsteils. Die Aufnahme 14 ist als eine Vertiefung in dem zweiten Formteil 13 ausgestaltet und weist auf ihrem Boden ein Loch 25 auf zum Hindurchführen von Steuerleitungen durch das zweite Formteil 13 hindurch, vgl. Fig. 7. Das zweite Formteil 13 weist außerdem ein zweites Rückhalteelement 15 auf, das als Außenkante des zweiten Formteils 13 ausgestaltet ist und dieses voll umfänglich umläuft. Die Aufnahme 14 wird von einem Dichtungselement 16 in Form eines Steges auf dem zweiten Formelteils 13 vollumfänglich umlaufen. Das hier gezeigte zweite Formteil 5 ist passend zu dem in den Fign. 2 bis 4b gezeigten ersten Formteil ausgestaltet und kann mit diesem lösbar verbunden werden, indem das erste Rückhalteelement 9 des erste Formteils 5 mit dem zweiten Rückhalteelement 15 des zweiten Formteils 13 in einen formschlüssigen Eingriff gebracht wird, vgl. Fign. 10a bis 10c. Die beiden Rückhalteelemente 9 und 15 sind beide aus einem elastischen Material wie etwa Silikon gefertigt, so dass die beiden Formteile 5 und 13 durch sie nicht nur zusammengehalten sondern auch aneinander gepresst werden, wodurch der erste flanschförmige Randbereich 10 des ersten Formteils mit einem zweiten flanschförmigen Randbereich 17 sich flächig berühren und aneinander gepresst werden. Auf diese Weise wird die Aufnahme 14 der Steuereinheit, die von den genannten beiden Randbereichen vollumfänglich umlaufen wird, sicher abgedichtet. Ferner wird das Dichtungselement 16 in die Ausnehmung 12 aufgenommen, wodurch ein zusätzlicher Dichtungseffekt erzielt wird.

In den Fign. 6 und 7 ist das in Fign. 5a und 5b gezeigte zweite Formteil in einer Ansicht von schräg oben bzw. von schräg unten schematisch dargestellt, wobei in der Aufnahme 14 des zweiten Formteils 13 nun eine Steuereinheit 18 angeordnet ist. Die Steuereinheit 18 weist eine erste Bedienungsschnittstelle 19 und eine zweite Bedienungsschnittstelle 20 auf, welche über das erste bzw. das zweite Bedienelement 3, 4 des ersten Formteils 5 bedient werden können, sobald die beiden Formteile miteinander verbunden sind, vgl. Fign. 10a bis 10c. Zu erkennen sind außerdem vier elektrische Steuerleitung 21, welche, wie in Fig. 7 dargestellt ist, durch das Loch 25 im zweiten Formteil hindurchgeführt sind. Ferner weist die Steuereinheit 18 eine Anzeige 22 auf zum Anzeigen eines Betriebs- oder Steuerzustandes der Steuereinheit in Abhängigkeit von einem mittels der Bedienschnittstellen 19, 20 eingestellten Steuerparameter. Dieser ist in dem vorliegenden Beispiel durch eine Heizleistung einer mit der Steuereinheit 18 verbindbaren und steuerbaren Heizeinrichtung gegeben, wie beispielsweise der in Fig. 1 gezeigten Heizvorrichtung 24.

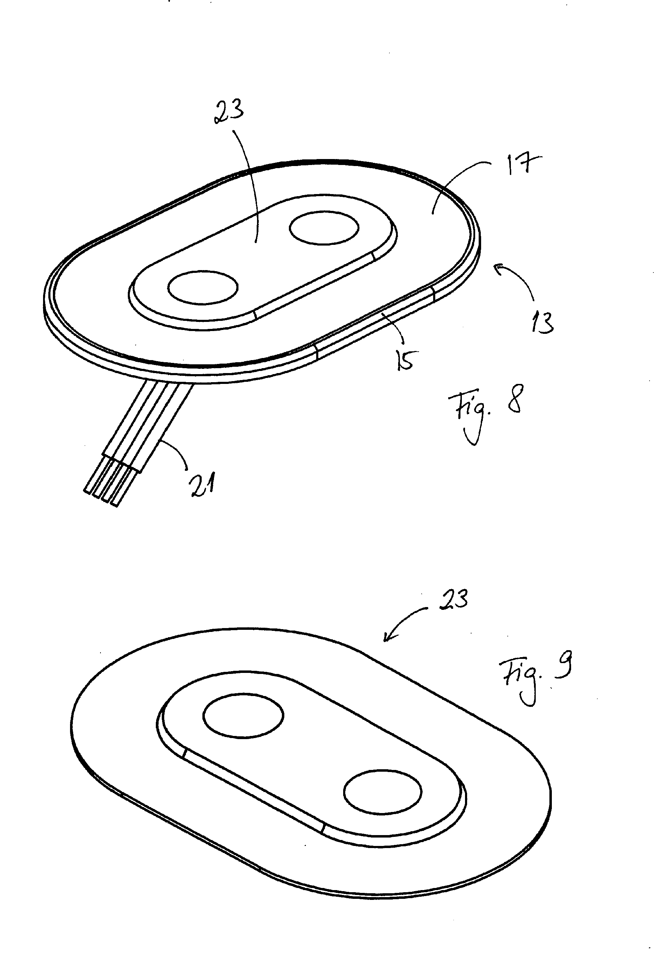

In Fig. 8 ist das in den Fign. 6 und 7 gezeigte zweite Formteil 13 mit einem Schutzdeckel 23 gezeigt, welches aus transparentem Silikon gefertigt ist und die Aufnahme 14 mit der in der Aufnahme 14 enthaltenen Steuereinheit 18 abschließt und auf diese Weise die Steuereinheit vor äußeren Einflüssen schützt, solange das zweite Formteil 13 nicht mit dem ersten Formteil 5 verbunden ist. In Fig. 9 ist dieser Schutzdeckel noch einmal in einer Detailansicht dargestellt, wobei die Steuerleitung mit einem ersten Ende mit der Steuereinheit 18 verbunden ist und an einem zweiten Ende eine Steckvorrichtung 26 aufweist zum Verbinden mit einer steuerbaren Vorrichtung.

In den Fign. 10a bis 10c sind die beiden Formteile 5 und 13 der vorangegangenen Fign. 2 und 8 getrennt (Fig. 10a bzw. Fig. 10b) und miteinander verbunden (Fig. 10c) schematisch dargestellt. Zum einen ist zu erkennen, dass die beiden Bedienschnittstellen 19 und 20 der Steuereinheit sich direkt unterhalb der beiden Bedienelemente 3 und 4 des ersten Formteils befinden und auf diese Weise manuell über die beiden Bedienelemente 3 und 4 bedient werden können. Zu erkennen ist außerdem, dass das krempenförmige erste Rückhalteelement 9 das zweite Rückhalteelement 15, also die Außenkante des zweiten Formteils 13, vollumfänglich hintergreift und auf diese Weise das erste Formteil 5 mit dem zweiten Formteil 13 formschlüssig verbindet. Dabei werden außerdem die beiden Formteile 5 und 13 mittels der beiden Rückhalteelemente 9 und 15 aneinander gepresst.

### Bezugszeichenliste

- 1: Kleidungsteil
- 2: Bedienteil
- 3: erstes Bedienelement
- 4: zweites Bedienelement
- 5: erstes Formteil
- 6: Naht
- 7: Außenhülle des Kleidungsteils
- 8: transparenter Bereich
- 9: erstes Rückhalteelement
- 10: erster Randbereich
- 11: Trageelement
- 12: Ausnehmung
- 13: zweites Formteil
- 14: Aufnahme
- 15: zweites Rückhalteelement
- 16: Dichtungselement
- 17: zweiter Randbereich
- 18: Steuereinheit
- 19: erste Bedienungsschnittstelle
- 20: zweite Bedienungsschnittstelle
- 21: Steuerleitung
- 22: Anzeige
- 23: Schutzdeckel
- 24: steuerbare Vorrichtung
- 25: Loch für Steuerleitung

## Patentansprüche

1. Kleidungsteil (1) mit einem Bedienteil (2) zum manuellen Bedienen einer mit dem Kleidungsteil (1) verbindbaren, steuerbaren Vorrichtung (24) mittels mindestens eines Bedienelementes (3, 4) des Bedienteils, wobei das Bedienteil (2) ein erstes Formteil (5) umfasst, welches fest mit dem Kleidungsteil (1) verbunden ist und welches das mindestens eine Bedienelement (3, 4) aufweist, und ein zweites Formteil (13), das eine Aufnahme (14) für eine Steuereinheit (18) der Vorrichtung (24) aufweist und mit dem ersten Formteil (5) verbindbar ist,
**dadurch gekennzeichnet,**
**dass** das erste Formteil (5) mindestens ein erstes Rückhalteelement (9) aufweist und dass das zweite Formteil (13) mindestens ein zweites Rückhalteelement (15) aufweist, wobei die beiden Rückhalteelemente (13, 15) in einen gegenseitigen Eingriff bringbar sind zum Herstellen einer lösbaren Verbindung zwischen dem ersten Formteil (5) und dem zweiten Formteil (13).

2. Kleidungsteil (1) nach Anspruch 1, **dadurch gekennzeichnet, dass** eines des mindestens einen ersten Rückhalteelements (9) als eine das erste Formteil (5) vollständig umlaufende Krempe ausgestaltet ist oder dass eines des mindestens einen zweiten Rückhalteelements (15) als eine das zweite Formteil (13) vollständig umlaufende Krempe ausgestaltet ist.

3. Kleidungsteil (1) nach einem der vorausgehenden Ansprüche, **dadurch gekennzeichnet, dass** eines des mindestens einen ersten Rückhalteelements (9) als eine Außenkante des ersten Formteils (5) ausgestaltet ist oder dass eines des mindestens einen zweiten Rückhalteelements (15) als eine Außenkante des zweiten Formteils (13) ausgestaltet ist.

4. Kleidungsteil (1) nach einem der vorausgehenden Ansprüche, **dadurch gekennzeichnet, dass** die Aufnahme (14) für die Steuereinheit (18) eine Vertiefung in dem zweiten Formteil (13) ist.

5. Kleidungsteil (1) nach einem der vorausgehenden Ansprüche, **dadurch gekennzeichnet**, **dadurch gekennzeichnet, dass** die Aufnahme (14) mit einer Schutzfolie oder einem Schutzdeckel (23) abgedeckt ist.

6. Kleidungsteil (1) nach einem der vorausgehenden Ansprüche, **dadurch gekennzeichnet, dass** das erste Formteil (5) einen flanschförmigen ersten Randbereich (10) aufweist und dass das zweite Formteil (13) einen flanschförmigen zweiten Randbereich (17) aufweist, wobei der erste Randbereich (10) und der zweite Randbereich (17) flächig aufeinanderliegen und die Aufnahme (14) vollständig umlaufen, wenn die beiden Formteile (5, 13) miteinander verbunden sind durch gegenseitigen Eingriff der beiden Rückhalteelemente (9, 15).

7. Kleidungsteil (1) nach einem der vorausgehenden Ansprüche, **dadurch gekennzeichnet, dass** das erste Formteil (5) und/oder das zweite Formteil (13) aus einem flexiblen Kunststoff gefertigt ist, insbesondere aus Silikon.

8. Kleidungsteil (1) nach einem der vorausgehenden Ansprüche, **dadurch gekennzeichnet, dass** das zweite Formteil (13) ein Loch (21) aufweist zum Hindurchführen von elektrischen Leitungen durch das zweite Formteil (13) hindurch.

9. Kleidungsteil (1) nach einem der vorausgehenden Ansprüche, **dadurch gekennzeichnet, dass** das erste Formteil (5) und/oder das zweite Formteil (13) ein Dichtungselement (16) aufweist, das die Aufnahme (14) vollständig umläuft und abdichtet, wenn die beiden Formteile (5, 13) miteinander verbunden sind durch gegenseitigen Eingriff der beiden Rückhalteelemente (9, 15).

10. Kleidungsteil (1) nach einem der vorausgehenden Ansprüche, **dadurch gekennzeichnet, dass** das erste Formteil (5) zumindest bereichsweise durchsichtig ausgestaltet ist für eine Sichtbarkeit einer Anzeige (22) der Steuereinheit.

11. Kleidungsteil (1) nach einem der vorausgehenden Ansprüche, **dadurch gekennzeichnet, dass** das Kleidungsteil (1) eine mit dem Kleidungsteil (1) verbundene Vorrichtung (24) umfasst sowie eine Steuereinheit (18) zum Steuern der Vorrichtung (24), wobei die Steuereinheit (18) in der Aufnahme (14) des zweiten Formteils (13) angeordnet ist.

12. Kleidungsteil (1) nach Anspruch 11, **dadurch gekennzeichnet, dass** die Steuereinheit (18) eine Steuerleitung (21) aufweist zum Übertragen von Steuersignalen auf die Vorrichtung (24), wobei die Steuerleitung (21) durch ein Loch (25) in dem zweiten Formteil (13) durch das zweite Formteil (13) hindurchgeführt und mit der Vorrichtung (24) verbindbar ist.

13. Kleidungsteil (1) nach einem der Ansprüche 11 oder 12, **dadurch gekennzeichnet, dass** die Steuereinheit (18) mindestens eine Eingabeschnittstelle (19, 20) aufweist zur manuellen Eingabe von Steuerparametern in die Steuereinheit (18), wobei die mindestens eine Eingabeschnittstelle (19, 20) mittels des mindestens einen Eingabeelement (3, 4) des ersten Formteils (5) bedienbar ist, wenn die beiden Formteile (5, 13) miteinander verbunden sind.

14. Kleidungsteil (1) nach einem der Ansprüche 11 bis 13, **dadurch gekennzeichnet, dass** es sich bei dem Kleidungsteil (1) um eine Jacke, eine Hose, einen Handschuh, einen Schuh, eine Socke, eine Mütze oder um einen Teil eines solchen Kleidungsteils handelt.

15. Kleidungsteil (1) nach einem der Ansprüche 11 bis 14, **dadurch gekennzeichnet, dass** die Vorrichtung (24) eine Heizvorrichtung für das Kleidungsteil (1) umfasst.
